(19) Europäisches Patentamt
European Patent Office
Office européen des brevets

(11) EP 3 763 698 B1

(12) **EUROPEAN PATENT SPECIFICATION**

(45) Date of publication and mention of the grant of the patent:
**29.11.2023 Bulletin 2023/48**

(21) Application number: **18908935.2**

(22) Date of filing: **05.10.2018**

(51) International Patent Classification (IPC):
***C07C 51/41*** (2006.01) ***B60C 1/00*** (2006.01)
***C07C 61/29*** (2006.01) ***C08K 5/098*** (2006.01)
***C08L 21/00*** (2006.01) ***C07F 15/06*** (2006.01)

(52) Cooperative Patent Classification (CPC):
(C-Sets available)
**C07C 51/412; B60C 1/00; C08K 5/098;**
**C08L 21/00;** C07F 15/06 (Cont.)

(86) International application number:
**PCT/JP2018/037465**

(87) International publication number:
**WO 2019/171643 (12.09.2019 Gazette 2019/37)**

(54) **COBALT SOAP, PRODUCTION METHOD THEREFOR, AND RUBBER BELT PRODUCED USING SAID COBALT SOAP**

KOBALTSEIFE, HERSTELLUNGSVERFAHREN DAFÜR UND UNTER VERWENDUNG DER BESAGTEN KOBALTSEIFE HERGESTELLTER GUMMIGURT

SAVON DE COBALT, SON PROCÉDÉ DE PRODUCTION, ET COURROIE EN CAOUTCHOUC PRODUITE À L'AIDE DUDIT SAVON DE COBALT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.03.2018 JP 2018042885**

(43) Date of publication of application:
**13.01.2021 Bulletin 2021/02**

(73) Proprietor: **Irec Co., Ltd.**
**Izumo-shi, Shimane 693-0043 (JP)**

(72) Inventor: **HARA, Takaharu**
**Izumo-shi, Shimane 693-0043 (JP)**

(74) Representative: **TBK**
**Bavariaring 4-6**
**80336 München (DE)**

(56) References cited:
**CN-A- 106 634 616**
**JP-A- S4 852 806**
**JP-A- H04 168 177**
**JP-A- S53 117 034**
**JP-A- S61 285 278**
**JP-A- S62 232 445**
**JP-A- S4 852 806**
**JP-A- H04 168 177**
**JP-A- H06 341 054**
**JP-A- S61 285 278**
**JP-A- S62 232 445**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 763 698 B1

(52) Cooperative Patent Classification (CPC): (Cont.)

C-Sets
**C07C 51/412, C07C 61/29**

## Description

### FIELD OF THE INVENTION

The present invention relates to a method for the production of cobalt soap, particularly an easy and cheap method for the production in high yield of high purity of cobalt soap. The invention also relates to a method for the production of a rubber belt using the cobalt soap and a use of the rubber belt.

### BACKGROUND OF THE INVENTION

Conventionally, cobalt soap has been widely used as an adhesion promotor for a steel cord and rubber. Adhesion of a steel cord and rubber is a technology necessary for producing, for example, belt component of a car tire or conveyor belt, etc. Cobalt soap is used as an adhesion promotor widely in the related technology field (Please refer to the following patent reference 1, non-patent references 1 and 2). Further, JP S62 232445 A aims to improve the adhesiveness and thermal cycling resistance of the obtained rubber composition by using a combination of cobalt resinate, at least one of resorcinol, [3-naphthol, etc., a maleimide derivative and silica as an adhesion promotor used for bonding a steel cord with a rubber.

The following metathesis method and direct method are known as methods for producing cobalt soap. For the metathesis method cobalt soap can be obtained by reacting cobalt chloride with sodium salts of a fatty acid (Please refer to the following non-patent reference 1).

The metathesis method can be represented by chemical formula $2RCOONa + CoCl_2 \rightarrow (RCOO)_2Co + 2NaCl$, wherein R represents any hydrocarbon.

For the direct method, cobalt soap can be obtained by the method for reaction of neutralizing cobalt hydroxide with a fatty acid (Please refer to the following non-patent reference 1).

The direct method can be represented by chemical formula $2RCOOH + Co(OH)_2 \rightarrow (RCOO)_2Co + 2H_2O$, wherein R represents any hydrocarbon.

Japan Patent Publication No. Sho. 60-158230
Japan Patent Publication No. Hei. 2-29097
Journal of JCCA, Vol. 3 (1966), pp. 20-24.
Nihon Rubber Kyokai Shi, Vol. 31 (1958), No. 5, pp. 380-391.

### SUMMARY OF THE INVENTION

Cobalt soap can be produced in high yield by the metathesis method. However, washing by water and then drying is necessary for the method because water and amount of salts remain in the reaction product, so the production cost is high as the result. In general production cost can be reduced by the direct method, but problems occur that depending on the kind of fatty acid as the material the reaction does not proceed smoothly, the reactant carbonizes under the circumstance at high temperature, the reaction does not complete, etc.

Further, since the specification of cobalt soap varies depending on the kind of fatty acid used as the material, cobalt soap is used properly by selecting a cobalt soap using a proper fatty acid for the production depending on the manufacturing product using cobalt soap, kind of steel cord or rubber compound, required property and specification of rubber, etc.

On the other hand, the softening point of cobalt soap is different depending on the kind of fatty acid as the material. In case the softening point of fatty acid is high, it may be a problem for the production of cobalt soap. Among fatty acids used for cobalt soap, rosin acid, tall oil fatty acid, naphthenic acid, stearic acid, palmitic acid, myristic acid, lauric acid, etc. can be listed as a fatty acid having a high softening point.

Among the fatty acids, particularly rosin acid has a high softening point. In case it is reacted by the direct method, it is necessary to use amount of organic solvent. Further, naphthenic acid is a mixture, so the softening point varies depending on the fatty acid included in the mixture. In general, naphthenic acid having a higher acid value has a higher softening point. The softening point of some fatty acid has a higher softening point than 100°C, so it is possible that the reaction by the direct method where an organic solvent is not used has a problem.

For the direct method mentioned above, a fatty acid as the raw material having a lower softening point has a lower viscosity, so the production becomes easier. It is necessary to stir the reactant to make it uniform, but an excess energy is needed when the viscosity of the reactant is high. In some case it is possible that a reaction apparatus such as a stirring blade. etc., can be damaged.

Further, water is produced as a byproduct by the reaction for the direct method. So, it is necessary to remove water by heating and reduced pressure. But, in case the viscosity of the reactant is high, water expands in the reactant

when it evaporates and the apparent volume of the reactant becomes huge. So, it is necessary to design the volume of reaction vessel large.

For the direct method, a method where the reaction temperature is raised high to reduce the viscosity of the reactant can be considered. But, it is possible in the method that the degradation in the quality of the product occurs because of the carbonization of the fatty acid, etc. Thus, the reaction temperature is limited to 230°C or less at the highest and desirably the reaction is done at 200°C or less

It can be considered that the viscosity is reduced by using a non-reactive organic solvent such as toluene or xylene, etc. in case such an organic solvent is used, cobalt soap having a high content of cobalt can be obtained. However, it is necessary to always remove the organic solvent by heating or reduced pressure after the reaction. Further, it is possible that the production cost becomes high and bad effects for the quality and human body occur because the organic solvent remains in the reactant.

As mentioned above, for the metathesis method and the direct method used for the production of cobalt soap, problems exist in the production cost, the quality of products, etc., the solution has been the problem to be solved.

Particularly, the problem exists that the production of cobalt rosinate (please refer to the patent reference 2), which is important among cobalt soaps, results in high cost compared to other cobalt soaps.

The inventors have been repeated various examinations with considering the above problems, then they found that cost can be reduced by adopting the direct method for the production of cobalt soap, and further that cobalt soap can be obtained in high yield and high purity by the production with using a softening agent for rubber blending without using an organic solvent such as toluene or xylene as the reaction solvent.

Accordingly, the purpose of the present invention is to provide a method for producing high yield and high purity of cobalt soap easily, cheaply, and in high quality. Further, the purpose of the present invention is to provide cheap and high quality of rubber belt, particularly, rubber belt with good adhesiveness of a steel cord and rubber, produced with using the cobalt soap.

Namely, the present invention is the following.

[1] A method for production of cobalt soap according to claim 1.
[2] The method for the production of a rubber belt according to claim 5.
[3] Use of a rubber belt according to claim 6.

According to the method for the production of the present invention, cobalt soap having a high purity can be cheaply and easily produced in high yield and quality. Further, cheap and good quality of rubber belt, particularly, rubber belt having good adhesiveness of a steel cord and rubber can be produced.

## BRIEF DESCRIPTION OF THE DRAWINGS

Figure 1 illustrates FTIR spectrum of cobalt soap of the present invention.

## DETAILED DESCRIPTION OF THE INVENTION

The detailed embodiment of the present invention is explained in the followings.

(1) Method for the production of cobalt soap

The method for the production of cobalt soap of the present invention is characterized in the method for the production of cobalt soap according to claim 1 to produce cobalt soap by reacting cobalt hydroxide with a fatty acid, wherein a softening agent for rubber blending is used as a reaction solvent. In the present invention, direct method, namely, method for obtaining cobalt soap by reacting cobalt hydroxide with a fatty acid is used. A softening agent for rubber blending is also used for the purpose of reducing the viscosity of cobalt soap during the reaction, etc.

Cobalt soap can be obtained in low cost, high yield, and high purity by using a softening agent for rubber blending as the reaction solvent without using a non-reactive organic solvent such as toluene or xylene. Non-reactive organic solvent such as toluene, xylene, etc., is generally used to reduce the viscosity during the reaction for producing cobalt soap. But, it is necessary to remove these solvents by heating or reduced pressure procedure during or after the chemical reaction. On the other hand, in general the softening agent for rubber blending used in the present invention has been a conventional ingredient blended in rubber, so it need not be removed like an organic solvent such as toluene or xylene by heating or reduced pressure and it can be used as an agent for rubber blending in the condition of the mixture with cobalt soap.

The softening agent for rubber blending is referred to as an agent blended in rubber for the purpose to improve work efficiency or processing performance by reducing the viscosity of low material compound or lowering the kneading

temperature at the step of the processing of rubber, or for the purpose to raise up the flexibility of rubber product after the production.

In case of the blending for the belt part of a tire and conveyer belt, about 5-15 parts of the softening agent for rubber blending is generally blended relative to 100 parts of rubber. Therefore, to use the softening agent for rubber blending not only erases concerns about the bad effect on the quality due to the residual when toluene or xylene is used, but leads improvement of the work environment and makes the removal by heating and reduced pressure unnecessary thereby resulting in cost savings.

For the softening agent for rubber blending which can be used, agents available for rubber blending, i.e. petroleum oils, coal tars, plant oils, are used. Agents having compatibility with cobalt soap, more desirably, also having compatibility with a fatty acid as the raw material, are preferable. The effect reducing the viscosity during the reaction of cobalt soap is obtained by using the softening agent for rubber blending. In case there is no compatibility between cobalt soap and the softening agent, the above effect is limited. In this case, the final product lacks uniformity and it is in the situation of the mixture of the softening agent as liquid and cobalt soap as solid.

A proper softening agent for rubber blending can be selected depending on the kind of cobalt soap to obtain the compatibility. For example, in case of cobalt rosinate obtained by the reaction of rosin as pine oil and cobalt hydroxide, a plant oil, particularly, pine tar is preferable because of the high compatibility.

For the petroleum oils, paraffin oils, aromatic oils, and naphthenic oils can be listed. In the present invention, petroleum oils are naphthenic oils. The average molecular weight is about 250 - 500. More specifically, paraffin, vaselin, petroleum resin, etc., can be listed.

For the coal tar oils, coal tar and coumarone-indene resin can be listed.

For the plant oils, stearic acid, lauric acid, cottonseed oil, soybean oil, peanut oil, pine oil, pine tar, rosin, etc., can be listed. In the present invention, plant oils are pine tar.

The pine tar is produced by fractionating tar among pine root oil and tar obtained by dry distilling pine root. Decomposition products of Cellulose and lignin, rosin, etc., are included as the main ingredients (please refer to non-patent reference).

In the present invention, a fatty acid having a high softening point (hereafter referred to "high softening point - fatty acid") is used for the production of cobalt soap. For the high softening point - fatty acid, rosin acid, tall acid, naphthenic acid, stearic acid, palmitic acid, myristic acid, lauric acid, etc., can be listed. In the present invention, the fatty acid having a high softening point is rosin acid.

The rosin acid is also called as resin acid and is obtained from the following three kinds of rosins. Gum rosin is obtained by purifying pine resin collected from pinaceae plants. Tall rosin is obtained by purifying tall oil produced as a byproduct of mainly paper industry. Wood rosin is obtained by purifying ingredients extracted with an organic solvent from old pine root.

For ingredients included in the rosin acid, abietic acid, dehydroabietic acid, parastolic acid, isopimaric acid, pimaric acid, etc., can be mainly listed (please refer to the following chemical formulae). The component ratio of them is not constant and differs depending on the kind of rosin as mentioned above, pine kind or origin of material, etc.

Chemical formula 1

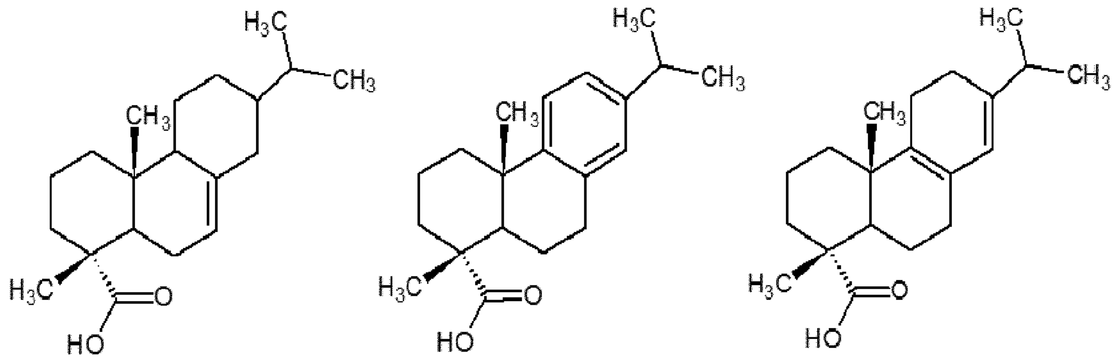

Abietic acid Dehydroabietic acid Parastolic acid

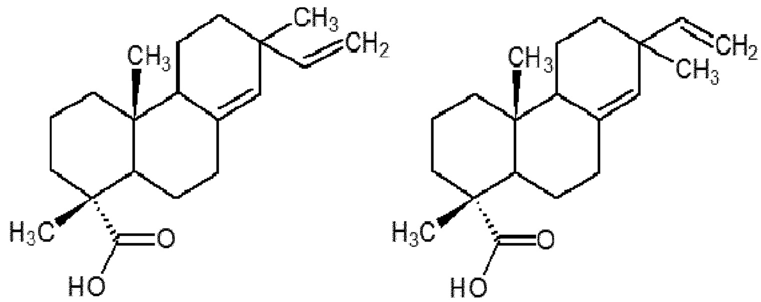

Isopimaric acid Pimaric acid

For the method of production of the present invention, the timing of the addition of the softening agent for rubber blending is not particularly limited, but time before the reaction by adding cobalt hydroxide is preferable. It is preferable that the reaction is carried out by adding cobalt hydroxide in the situation a fatty acid as a raw material and a softening agent are mixed or dispersed in advance.

Water is generated as a byproduct by neutralization reaction of cobalt hydroxide with a fatty acid. Water can be removed by heating and procedure under reduced pressure. In case the viscosity during the reaction is high, water tends to accumulate in the reactant, but the removal of water can be smoothly proceeded by reducing the viscosity. Heating must be at 230°C at the highest, 200°C or less is preferable.

(2) Cobalt soap

The Cobalt soap of the present invention is produced as above and the content (weight ratio) of metal cobalt of the cobalt soap is 4.0% - 6.0% or more.

(3) Rubber belt

The method for producing a rubber belt of the present invention is, as above, characterized by using the cobalt soap produced by reacting cobalt hydroxide with a fatty acid as an adhesion promotor between a steel cord and rubber. The cobalt soap is used as an adhesion promotor in the rubber compound for the adhesion to the steel cord.

For the rubber belt, belt part of a car tire, a conveyer belt, a rubber hose, etc., can be listed.

A steel cord and rubber can be adhered by using rubber compound including cobalt soap. Thus, the performance of a rubber belt can be evaluated by the adhesive performance between rubber compound, steel cord, and rubber.

A car tire of the present invention is produced by using the rubber belt as the belt part.

## Examples

The present invention is explained below in detail with examples listed, but the present invention should not be limited by the examples.

(1) Measurement of the cobalt content in cobalt soap

Cobalt soap was precisely weighed in an Erlenmeyer flask, 40 mL of sulfonic acid and boiling bubble stones are added, then the mixture was heated. The cobalt soap was completely decomposed to obtain an acid solution of cobalt soap. The acid solution was filtered with a filter paper to remove the oil content. Then, MX indicator was added and 1N of ammonia water was added until the color changed to yellow. After the color changed to yellow, the solution was titrated with 0.05 mol. of EDTA ▪ 2Na titration solution. When the yellow color changes to orange yellow or red, ammonia water solution was added. Titration was done with checking the change of the color. Titration was also done with repeating to drip, titrate, and stir the ammonia water. Then, the content of metal was calculated according to the following formula 1.

Chemical formula 1

$$\text{Content of Cobalt (\%)} = \frac{A \times F \times 2.947}{\text{Amount of sample (mg)}}$$

A : 0.05mol/ L Volume of EDTA/2Na Solution used (ml)

F : 0.05mol/ L Titer of EDTA/2Na Solution

Example 1

In a 2000 mL volume of Erlenmeyer glass flask, placed 497 g of rosin acid (170 of acid value) commercially available (manufactured by Harimaya Chamical Co., Ltd.) and 331 g of pine tar, one of plant oils, commercially available (manufactured by Tokyo Resin Industry Co., Ltd.), then the mixture was melted by heating in a mantle heater. After the rosin acid was melted, it was stirred at a rate of 300 rpm by a stirring wing to be completely mixed. The mixture was heated to 140 °C and then 70 g of cobalt hydroxide was added little by little with being careful for the reactant not to overflow from the flask because of the expansion of reactant water. The reactant was heated to 200 °C with removing the water. Then, the reactant was reduced pressure to remove 22 g of water and 15 g of oil content and the product was obtained. The product obtained above had a 5.07 % content of cobalt (including pine tar).

Reference Example 2

In a 2000 mL volume of Erlenmeyer glass flask, placed 497 g of rosin acid (170 of acid value) commercially available (manufactured by Harimaya Chamical Co., Ltd.) and 331 g of SNH220, one of plant oils, commercially available (manufactured by Sankyo Yuka Industry Co., Ltd.), then the mixture was melted by heating in a mantle heater. After the rosin acid was melted, it was stirred at a rate of 300 rpm by a stirring wing to be completely mixed. They were heated to 130 °C and then 70 g of cobalt hydroxide was added little by little with being careful for the reactant not to overflow from the flask because of the expansion of reactant water. The reactant was heated to 200 °C with removing the water. Then, the reactant was reduced pressure to remove 27 g of water. The product obtained above (including SNH220) had a 4.99 % content of cobalt. The product was not in a uniformed situation but in a mixed situation of liquid and solid.

Example 3

In a 2000 mL volume of Erlenmeyer glass flask, placed 461 g of rosin acid (170 of acid value) commercially available (manufactured by Harimaya Chamical Co., Ltd.), 56 g of pine tar, one of plant oils, commercially available (manufactured by Tokyo Resin Industry Co., Ltd.), and 252 g of coumarone resin L-5, one of tar softening agents (manufactured by Nitto Chemical Co., Ltd.), then the mixture was melted by heating in a mantle heater. After the rosin acid was melted, it was stirred at a rate of 300 rpm by a stirring wing to be completely mixed. They were heated to 130 °C and then 65 g of cobalt hydroxide was added little by little with being careful for the reactant not to overflow from the flask because of the expansion of reactant water. The reactant was heated to 200 °C with removing the water. Then, the reactant was reduced pressure to remove 28 g of water. The product obtained above had a 5.01 % content of cobalt (including pine tar). The product was not in a uniformed situation but in a mixed situation of liquid and solid.

Comparative Example 1

In a 2000 mL volume of Erlenmeyer glass flask, placed 1065 g of rosin acid (170 of acid value) commercially available (manufactured by Harimaya Chamical Co., Ltd.), then it was melted by heating in a mantle heater. After the rosin acid was melted, it was stirred at a rate of 300 rpm by a stirring wing. They were heated to 140 °C and then 150 g of cobalt hydroxide was tried to add little by little with being careful for the reactant not to overflow from the flask because of the expansion of reactant water. However, at the time 50 g of cobalt hydroxide was added, the reactant got hard, so the reaction did not proceed.

FTIR (Fourier Transform Infrared Spectrophotometer) spectra of law material rosin acid, cobalt soap obtained Example 1 (including solvent), and cobalt soap produced by the known method for production are shown in Figure 1.

Comparing cobalt soap of Example 1 (2) in Figure 1 with cobalt soap produced by the known method for production (3), FTIR spectra of them match. Accordingly, the examples suggest that rosin acid was obtained.

According to Examples 1 and 3 and Reference Example 2, cobalt soap having a high purity of cobalt can be produced by the method of production using a softening agent for rubber blending of the present invention. Conventionally it is said that the purity of cobalt of cobalt soap is 7% to 8% in case of cobalt rosinate is used. Considering that the product includes a softening agent for rubber blending such as pine tar, etc., the cobalt rosinate of the present invention has a purity of cobalt comparable to the conventional cobalt rosinate. Thus, cobalt rosinate having an allowable purity can be easily obtained in lower cost by the method of production of the present invention.

According to Examples 1 and 3 and Reference Example 2, it is elucidated that rosin acid has a good compatibility with plant oils, especially preferable compatibility with pine tar.

Thus, it is preferable to use these softening agents for producing cobalt rosinate.

(2) Evaluation of the adhesiveness of a steel cord and rubber with rubber compound including cobalt rosinate

Preparation of rubber compound

Rubber compounds blended as shown in Table 1 were prepared by mixing with a banbury mixer (Examples 4-7 and Comparative Examples 2 - 5). A mixture including compositions other than vulcanization system-composition was added to a polymer (NR: natural rubber CV60) at 20 RPM of rotor rotation speed for the first step of mixing, further another composition was added at 40 RPM rotor rotation speed, kneaded for 60 seconds, and released at 160 °C. A vulcanization system-composition was mixed at 30 RPM of rotor rotation speed at 100 °C and the composition was shaped in a sheet shape by a roll.

The conditions for making an adhesive strength specimen and for the adhesiveness test are the followings and some kinds of performance tests (Anti-heat aging test and High temperature and high humidity test) were carried out.

Conditions for making an adhesive strength specimen

A brass painting steel cord (1 × 2 × 0.30 HT) manufactured by Tokyo Seiko Co., Ltd. was used. In the steel cord, the % content of Cupper in brass was 63.0 %. The brass plating steel cords adjusted in a length of about 200 mm were arranged parallel at about 25 mm of intervals, the boss sides of them were pinched with rubber compounds, and press vulcanized in the following conditions. Then, the sample was cut so that the embedded length of the steel cord is about 7 mm to make an adhesive strength test specimen. The condition of press vulcanization was 160°C × 15 min.

Adhesion test

Adhesive strength test was performed according to ASTM D2229.
ASTM D2229: 2014 "Standard Test Method for Adhesion of Steel Tire Cords and Rubber"
Shape of test specimen: 12.5 mm × 15 mm × adhesion length about 7 mm
Steel coed: Brass plating steel cord 1 × 2 × 0.3 HT (manufactured by Tokyo Seiko Co., Ltd.)
Test speed: Adhesive strength (Pull-out force) and rubber coverage (%; visually) were evaluated when a cord is pulled out from a rubber/cord sample at a speed of 100 mm/min.
Test number: 5 test specimens for each test
Test temperature: 23°C
Test machine: Autograph (AG-Xplus 10kN, manufactured by Shimazu Seishakusho Co., Ltd.)

Anti-heat aging test

For an adhesion test sample, pull-out force test was carried out after the treatment at 70°C for 168 hours, and adhesive strength (Pull-out force) and rubber coverage were evaluated.

High temperature high humid test

For an adhesion test sample, pull-out force test was carried out after the treatment at 70°C × 95% RH for 168 hours, and adhesive strength (Pull-out force) and rubber coverage were evaluated.

The adhesion test results of the test specimens, which were made based on the blending of the rubber compound in Table 1, are shown in table 2.

For the above blending, 100 weight parts of NR (natural rubber: CV60), 60 weight parts of carbon black (SEAST 3M), 1 part of Anti-aging agent (Ozonone 6c), 10 parts of 3 kinds of Zinc flower (manufactured by Hakusui tech Co., Ltd.), 1 part of stearic acid, 7 parts of insoluble sulfur (20 % oil) as a vulcanizing agent were used. The speeds of the following 4 kinds with different vulcanizing speeds as the vulcanizing agent were compared.

Vulcanizing agent

CZ (N-Cychlohexyl-2-benzothiazole sulfenamide): 0.35
NS (N-Oxydiethylene-2-benzothiazole sulfenamide): 0.33
DM (2-benzothiazyl disulfide): 0.44
TBSI (N-t-Butyl-2-benzothiazolyl sulfenimide): 0.53

Each vulcanizing agent is different in its molecular weight, so each blending amount is adjusted so that the same MOL of each agent is added.

In Examples 4 - 7 of Table 1, 6.35 % of cobalt rosinate in pine tar was used.

In Comparative Examples 2-5, cobalt rosinate including no pine tar and 7.4 % cobalt was used. 3.14 phr was included in Examples 4 - 7 and 2.70 phr was included in Comparative Examples 2-5, wherein the elemental amount of cobalt in the rubber compound was adjusted to 0.2 phr. Here, "phr" means the amount of each ingredient relative to 100 parts of law material rubber (parts per hundred parts by weight of rubber).

| | | | | | | | | | | |
|---|---|---|---|---|---|---|---|---|---|---|
| | | | | | | | | | | |
| | Table 1 Blending of rubber compound | | | | | | | | | |
| | | | | | | | | | | |
| | Example | 4 | 5 | 6 | 7 | | | | | |
| | Comparative Example | | | | | 2 | 3 | 4 | 5 | |
| | | | | | | | | | | |
| | NR CV60 | 100 | 100 | 100 | 100 | 100 | 100 | 100 | 100 | |
| | Carbon HAF | 60 | 60 | 60 | 60 | 60 | 60 | 60 | 60 | |
| | | | | | | | | | | |
| | Cobalt rosinate 6.35% (includes pine tar) | 3.14 | 3.14 | 3.14 | 3.14 | | | | | |
| | Cobalt rosinate 7.496 | | | | | 2.70 | 2.70 | 2.70 | 2.70 | |
| | Anti-aging agent 6C | 1 | 1 | 1 | 1 | 1 | 1 | 1 | 1 | |
| | Zinc oxide ZnO | 10 | 10 | 10 | 10 | 10 | 10 | 10 | 10 | |
| | Stearic acid | 1 | 1 | 1 | 1 | 1 | 1 | 1 | 1 | |
| | | | | | | | | | | |
| | (Vulcanization system) | | | | | | | | | |
| | Insoluble sulfur (20% oil) | 7 | 7 | 7 | 7 | 7 | 7 | 7 | 7 | |

(continued)

| | | | | | | | | |
|---|---|---|---|---|---|---|---|---|
| (Vulcanization system) | | | | | | | | |
| CZ | 0.35 | | | | 0.35 | | | |
| NS | | 0.33 | | | | 0.33 | | |
| DM | | | 0.44 | | | | 0.44 | |
| TBSI | | | | 0.53 | | | | 053 |
| | | | | | | | | |

| | | | | | | | | |
|---|---|---|---|---|---|---|---|---|
| | | | | | | | | |
| Table 2 Results of Adhesiveness test | | | | | | | | |
| | | | | | | | | |
| Example | 4 | 5 | 6 | 7 | | | | |
| Comparative Example | | | | | 2 | 3 | 4 | 5 |
| | | | | | | | | |
| Initial adhesiveness (N/mm) | 29.6 | 34.5 | 36.5 | 39.9 | 28.9 | 37.5 | 35.6 | 38 |
| Rubber coverage (%) | 95 | 96 | 95 | 91 | 92 | 94 | 94 | 88 |
| | | | | | | | | |
| Anti-heat aging adhesiveness (N/mm) | 32.2 | 39.8 | 39.4 | 39.0 | 403 | 38.8 | 358 | 37.1 |
| Rubber coverage (%) | 95 | 95 | 95 | 95 | 95 | 94 | 95 | 92 |
| | | | | | | | | |
| High temperature High humid adhesiveness (N/mm) | 26.5 | 25.7 | 28.8 | 27.8 | 246 | 24.7 | 26.5 | 23.1 |
| Rubber coverage (%) | 75 | 54 | 83 | 70 | 72 | 51 | 68 | 34 |
| | | | | | | | | |
| | | | | | | | | |

As the result of adhesion test in Table 2, cobalt rosinate with and without pine tar are both good in the adhesiveness for the initial adhesiveness and heat aging adhesiveness. For high temperature high humidity adhesiveness, Examples 4 - 7 with pine tar were evaluated better than Comparative Examples 2-5 without pine tar.

It was found that the present invention has an advantage of cobalt rosinate with pine tar from the viewpoint of the synthesis. Further, it was elucidated that the adhesiveness of a steel cord and rubber is good in the performance.

## Claims

1. A method for the production of cobalt soap which produces cobalt soap by reacting cobalt hydroxide with a fatty acid, wherein a softening agent for rubber blending is used as the reaction solvent, wherein the softening agent for rubber blending is at least one selected from the group consisting of petroleum oils, coal tar oils, and plant oils, wherein the fatty acid is a fatty acid having a high softening point and the fatty acid having a high softening point is rosin acid, and wherein the petroleum oils are naphthenic oils and the plant oils are pine tar.

2. The method for the production according to claim 1, wherein pine tar is used as the reaction solvent.

3. The method for the production according to claim 1 or 2, wherein no reaction solvent except for pine tar, naphthenic oils, or coal tar is used.

4. The method for the production according to any one of claims 1 - 3, wherein the rosin acid is one derived from any

one selected from the group consisting of gum rosin, tall rosin, and wood rosin.

5. A method for the production of a rubber belt produced by using a cobalt soap as an adhesion promotor of a steel cord and rubber, wherein the cobalt soap is produced according to the method defined in any one of claims 1 - 4.

6. Use of a rubber belt produced according to claim 5 for any one selected from the group consisting of belt portion for a car tire, a conveyer belt and a rubber hose.

## Patentansprüche

1. Verfahren zur Herstellung von Cobaltseife, das Cobaltseife durch Umsetzen von Cobalthydroxid mit einer Fettsäure herstellt, wobei ein Weichmacher für die Kautschukmischung als das Reaktionslösungsmittel verwendet wird, wobei der Weichmacher für die Kautschukmischung mindestens einer ist, der aus der Gruppe ausgewählt ist, die aus Erdöl, Kohlenteerölen und Pflanzenölen besteht, wobei die Fettsäure eine Fettsäure mit einem hohen Erweichungspunkt ist und die Fettsäure mit einem hohen Erweichungspunkt Kolophonium-Säure ist, und wobei die Petroleumöle Naphthenöle sind und die Pflanzenöle Kiefernteer sind.

2. Verfahren zur Herstellung nach Anspruch 1, wobei Kiefernteer als das Reaktionslösungsmittel verwendet wird.

3. Verfahren zur Herstellung nach Anspruch 1 oder 2, wobei kein anderes Reaktionslösungsmittel als Kiefernteer, Naphthenöle oder Kohlenteer verwendet wird.

4. Verfahren zur Herstellung nach einem der Ansprüche 1 - 3, bei dem die Kolophonium-Säure von einem der folgenden Stoffe abgeleitet ist: Gummikolophonium, Tallkolophonium und Holzkolophonium.

5. Verfahren zur Herstellung eines Kautschukriemens, der unter Verwendung einer Cobaltseife als Haftvermittler zwischen einem Stahlcord und Kautschuk hergestellt wird, wobei die Cobaltseife nach dem in einem der Ansprüche 1 - 4 definierten Verfahren hergestellt wird.

6. Verwendung eines nach Anspruch 5 hergestellten Kautschukriemens für einen ausgewählt aus der Gruppe bestehend aus einem Gürtelabschnitt für einen Autoreifen, ein Förderband und einen Gummischlauch.

## Revendications

1. Méthode de production de savon de cobalt, qui produit du savon de cobalt en faisant réagir de l’hydroxyde de cobalt avec un acide gras, dans laquelle un plastifiant pour le mélange de caoutchouc est utilisé comme solvant de réaction, où le plastifiant pour le mélange de caoutchouc est au moins un plastifiant choisi dans le groupe constitué par les huiles de pétrole, les huiles de goudron de houille, et les huiles végétales, où l’acide gras est un acide gras ayant un point de ramollissement élevé et l’acide gras ayant un point de ramollissement élevé est l’acide résinique, et où les huiles de pétrole sont des huiles naphténiques et les huiles végétales sont constituées de goudron de pin.

2. Méthode de production selon la revendication 1, dans laquelle du goudron de pin est utilisé comme solvant de réaction.

3. Méthode de production selon la revendication 1 ou 2, dans laquelle aucun solvant de réaction autre que le goudron de pin, les huiles naphténiques, ou le goudron de houille, n’est utilisé.

4. Méthode de production selon l’une quelconque des revendications 1-3, dans laquelle l’acide résinique est un acide dérivé de l’un quelconque choisi dans le groupe constitué par la colophane de gomme, la colophane de tallöl et la colophane de bois.

5. Méthode de production d’une courroie en caoutchouc produite à l’aide d’un savon de cobalt comme promoteur d’adhésion d’un câble d’acier et d’un caoutchouc, dans laquelle le savon de cobalt est produit selon la méthode définie selon l’une quelconque des revendications 1-4.

6. Utilisation d’une courroie en caoutchouc produite selon la revendication 5, pour l’un(e) quelconque choisi(e) dans

le groupe constitué par une portion de courroie pour un pneu de voiture, une courroie transporteuse et un tuyau en caoutchouc.

Figure 1

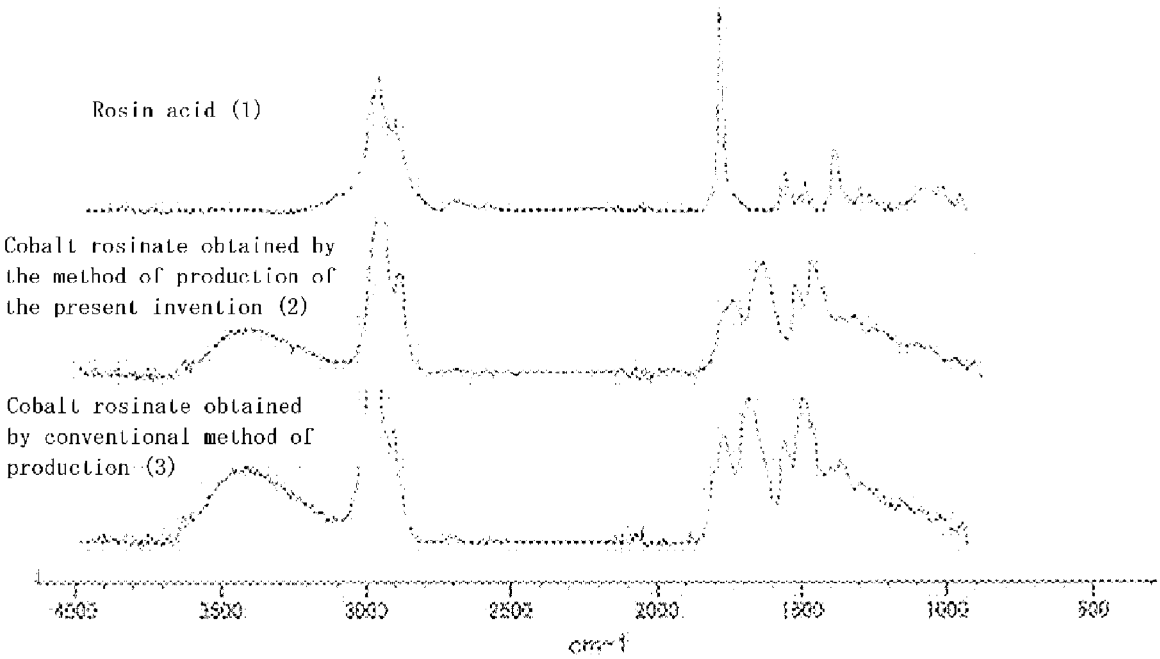


FTIR Spectra of Cobalt rosinate

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S62232445 A
- JP SHO60158230 A
- JP HEI229097 A


### Non-patent literature cited in the description


- *Journal of JCCA,* 1966, vol. 3, 20-24
- *Nihon Rubber Kyokai Shi,* 1958, vol. 31 (5), 380-391